# EUROPÄISCHE PATENTANMELDUNG

(11) **EP 0 987 028 A1**
(43) Veröffentlichungstag der Anmeldung: **22.03.2000**
(21) Anmeldenummer: 98114851.3
(22) Anmeldetag: 07.08.1998
(51) Int. Cl.: A61K 38/17

(54) **Pharmazeutische Zusammensetzung von Hedgehog-Proteinen und deren Verwendung**

(71) Anmelder: F. HOFFMANN-LA ROCHE AG, 4070 Basel (CH)
(72) Erfinder: Die Erfindernennung liegt noch nicht vor
(74) Vertreter: Schreiner, Siegfried, Dr.

(57) **Zusammenfassung**

Eine pharmazeutische Zusammensetzung eines Hedgehog-Proteins, welches als Zusatzstoff Zinkionen, Magnesiumionen, Calciumionen, Sulfationen, Cyclodextrin und/oder Heparin enthält, ist insbesondere bei Raumtemperatur stabil.

## Beschreibung

Gegenstand der Erfindung ist eine pharmazeutische Zusammensetzung von Hedehog-Proteinen und deren Verwendung.

Unter Hedgehog (hh)-Proteinen versteht man eine Familie von sekretierten Signalproteinen, die verantwortlich sind für die Ausbildung einer Vielzahl von Strukturen in der Embryogenese (J.C. Smith, Cell 76 (1994) 193 - 196, N. Perrimon, Cell 80 (1995) 517 - 520, C. Chiang et al., Nature 83 (1996) 407, M.J. Bitgood et al., Curr. Biol. 6 (1996) 296, A. Vortkamp et al., Science 273 (1996) 613, C.J. Lai et al., Development 121 (1995) 2349). Bei der Biosynthese wird nach Abspaltung der Signalsequenz und autokatalytischer Spaltung eine 20 kD N-terminale Domäne und eine 25 kD C-terminale Domäne erhalten. Die N-terminale Domäne ist in ihrer natürlichen Form Cholesterol-modifiziert (J.A. Porter et al., Science 274 (1996) 255 - 259). In höheren Lebewesen besteht die hh-Familie zumindest aus drei Mitgliedern, nämlich Sonic, Indian und Desert hh (Shh, Ihh, Dhh; M. Fietz et al., Development (Suppl.) (1994) 43 - 51). Für Hedgehog-Proteine, welche rekombinant hergestellt wurden, wurde eine unterschiedliche Aktivität nach Herstellung in Prokaryonten und Eukaryonten beobachtet (M. Hynes et al., Neuron 15 (1995) 35 - 44 und T. Nakamura et al., Biochem. Biophys. Res. Comm. 237 (1997) 465 - 469.

Hynes et al. vergleichen die Aktivität von hh im Überstand von transformierten "human embryonic kidney 293 cells" (eukaryontischer hh) mit aus E.coli hergestellten hh und finden eine vierfach höhere Aktivität des hh aus den Überständen der Nierenzellinie. Als Ursache dieser erhöhten Aktivität wird ein potentieller zusätzlicher "accessory factor", der nur in eukaryontischen Zellen exprimiert wird, eine posttranslationale Modifikation, ein unterschiedlicher N-Terminus, der aus E.coli isolierte hh enthält 50 % eines hhs, welcher zwei zusätzliche N-terminale Aminosäuren (Gly-Ser) trägt, oder um 5 - 6 Aminosäuren verkürzt ist, oder in einem höheren Aggregatzustand (z. B. durch Bindung an Nickel-Agarose beads) diskutiert.

Nakamura et al. vergleichen die Aktivität von shh im Überstand von transformierten "chicken embryo fibroblasts" mit einem aus E.coli isolierten shh Fusionsprotein, das noch einen N-terminalen Polyhistidinteil aufweist. Das shh im Überstand der Fibroblasten hat bezüglich der Stimulation von alkalischer Phosphatase (AP) in C3H10T ½ Zellen eine 7-fach höhere Aktivität als das gereinigte E.coli Protein. Als Ursache der erhöhten Aktivität werden Moleküle, wie beispielsweise bone morphogenetic Proteins (BMPs) diskutiert, die nur im Überstand von eukaryontischen Zellen vorhanden sind und die stärkere Induktion der AP verursachen.

Pepinski et al. (J. Biol. Chem. 273 (1998) 14037 - 14045) haben eine shh-Form, welche mit Palmitinsäure modifiziert ist, identifiziert. Diese shh-Mutante ist 30fach potenter als die unmodifizierte Form im C3H10T ½ Assay.

Von Kinto et al., FEBS Letters, 404 (1997) 319 - 323 wurde beschrieben, daß hh sekretierende Fibroblasten bei i.m. Implantation auf Collagen eine ektopische Knochenbildung induzieren. Hedgehog-Proteine besitzen also eine osteoinduktive Aktivität.

Aus Yang et al., Development 124 (1997) 4393-4404, ist bekannt, daß für eine pharmazeutisch wirksame in vivo Aktivität am Wirkort im Körper hohe lokale Hedgehog-Konzentrationen über einen Zeitraum von mindestens 16 h vorherrschen müssen. Das von Yang et al. beschriebene Trägersystem wie das Hedgehog-beladene Chromatographie-Medium Affigel CM, die von Marti et al. in Nature 375 (1995) 322-325 beschriebene Ni-Agarose oder das von Lopez-Martinez et al. in Curr. Biol. 5 (1995) 791-796 verwendete Affigel Blue oder die darin verwendeten Heparin-Agarose-Partikel sind für eine pharmazeutische Anwendung wenig geeignet, da sie immunogen sind und entzündliche Reaktionen hervorrufen können.

Aufgabe der Erfindung ist es, eine stabile, vorzugsweise wässrige pharmazeutische Zusammensetzung eines Hedgehog-Proteins zur Verfügung zu stellen.

Die Aufgabe wird gelöst durch eine pharmazeutische Zusammensetzung eines Hedgehog-Proteins, welche ein Hedgehog-Protein in einer pharmazeutisch wirksamen Menge und als Zusatzstoff Zinkionen, Magnesiumionen, Calciumionen, Sulfationen, Cyclodextrin und/oder Heparin enthält.

Es hat sich überraschenderweise gezeigt, daß ein oder mehrere Zusatzstoffe, ausgewählt aus der Gruppe Zinkionen, Magnesiumionen, Sulfationen und Heparin, in der Lage ist, Hedgehog-Proteine (als pharmazeutische Zusammensetzung und in sonstiger Form), vorzugsweise in wässriger Lösung zu stabilisieren. Dadurch kann beispielsweise bei Raumtemperatur die Aktivität des Hedgehog-Proteins über lange Zeit erhalten werden, und die pharmazeutischen Zusammensetzungen von Hedgehog-Proteinen sind über lange Zeit bei Raumtemperatur lagerbar. Die erfindungsgemäßen Zusatzstoffe sind aber auch zur Stabilisierung von Hedgehog-Lyophilisaten (bulk oder pharmazeutische Zusammensetzung) geeignet und stabilisieren die Hedgehog-Proteine auch während der Herstellung von Hedgehog-Präparaten, wie Implantaten, Micropartikeln, Gelen etc..

In der erfindungsgemäßen wässrigen Lösung des Hedgehog-Proteins liegt der Zusatzstoff in einem molaren Überschuß bezüglich der Hedgehog-Proteine vor. Dieser Überschuß ist vorzugsweise 1 - 100fach. Die wässrige erfindungsgemäße Lösung ist insbesondere zur Herstellung von Kombinationen von Hedgehog-Proteinen mit Trägerstoffen geeignet.

Ein weiterer Gegenstand der Erfindung ist deshalb eine wässrige Lösung eines Hedgehog-Proteins, welche dadurch gekennzeichnet ist, daß sie einen erfindungsgemäßen Zusatzstoff in einem molaren Überschuß bezüglich des Hedgehog-Proteins enthält. Diese Lösung ist vorzugsweise gepuffert und/oder lyophilisiert.

Die Menge der erfindungsgemäßen Zusatzstoffe ist an sich unkritisch und in einem weiten Bereich variierbar. Die geeigneten Mengen richten sich nach der pharmazeutischen Verträglichkeit des Zusatzstoffes und des Umfanges der stabilisierenden Wirkung bei einer pharmazeutisch akzeptablen Konzentration. Zinkionen sind als Stabilisator besonders bevorzugt und können beispielsweise vorteilhaft in einer Konzentration von 0,01 - 100 mmol/l zugesetzt werden. Bei dieser Konzentration zeigt sich ein deutlicher stabilisierender Effekt auf die Hedgehog-Proteine. Solche Zinkionenkonzentrationen sind pharmazeutisch noch verträglich.

Cyclodextrin kann erfindungsgemäß vorzugsweise als Cyclodextrinsulfat vorliegen. Die Konzentrationen liegen bevorzugt zwischen 1 und 20 Gew.-%. Als Heparin wird vorzugsweise niedermolekulares Heparin (ca. 3 kDa) verwendet. Die Konzentration beträgt vorzugsweise 0,5 - 50 mg/ml für niedermolekulares Heparin, für höhermolekulares Heparin werden entsprechende molare Mengen verwendet. Sulfationen werden vorzugsweise als Zinksulfat eingesetzt. Die Konzentration der Sulfationen beträgt vorzugsweise 0,01 - 100 mmol/l. Calcium- und Magnesiumionen werden vorzugsweise in einer Konzentration von 0,01 - 100 mmol/l eingesetzt.

Vorzugweise ist das Hedgehog-Protein dabei auf einen biokompatiblen Träger lokalisiert, wobei der Träger das Hedehogprotein in der aktiven, gefalteten Struktur bindet und lokal in vivo in aktiver Form verzögert freisetzen kann. Derartige Formulierungen sind insbesondere für die Reparatur von Knochen- und Knorpeldefekten geeignet, können aber auch für die Reparatur neuronaler Defekte oder für ein systemisches Delivery verwendet werden.

In einer bevorzugten Ausführungsform enthält die pharmazeutische Zusammensetzung das Hedgehog-Protein gebunden an einen hydrophilen Träger, der biokompatibel ist und beispielsweise als Implantat eingesetzt werden kann. Vorzugsweise ist der Träger eine Polymer, welches
- das Hedgehog-Protein aufgrund von ionischen Wechselwirkungen als negativ geladener Träger bindet,
- das Hedgehog-Protein bei der Bindung an den Träger nicht denaturiert wird,
- der Träger unter neutralen Bedingungen mindestens 0,1 bis 1, vorzugsweise 0,1 bis 2 negativ geladene Reste pro Monomer enthält,
- die Ladung vermittelt in Form von Säuregruppen wie z.B. Sulfat-, Carboxyl-oder Phosphatgruppen,
- und das durchschnittliche Molekulargewicht des Trägers mindestens 50.000 Da ist.

Es hat sich gezeigt, daß Hedgehog-Proteine dann reversibel, aktiv und verzögert von einem Träger in vivo freigesetzt werden, wenn sie an eine negativ geladene, lösliche oder unlösliche Polymermatrix gebunden werden. Solche Trägermatrices sind beispielsweise in der europäischen Patentanmeldung Nr. 98104416.7 beschrieben.

Unter pharmazeutischer Wirkung wird vorzugsweise eine neurologische Wirkung auf Nervenzellen, eine Chondrogenese und/oder -induktion und vorzugsweise Osteogenese und/oder -induktion verstanden, wie sie in Kinto et al., FEBS Letters, 404 (1997) 319-323 für die Knocheninduktion, für die Wirkung auf Nervenzellen durch Miao et al. in J. Neurosci. 17 (1997) 5891-5899 und für die Knorpelzelleninduktion von Stott et al. in J. Cell Sci. 110 (1997) 2691-2701 beschrieben ist.

Zur Herstellung von Trägermatrices, die mit Hedgehog-Proteinen in einer solchen Weise beschichtet sind, daß sie bei einer Lokalapplikation eine ausreichende pharmazeutische Wirksamkeit zeigen, sind Lösungen der Hedgehog-Proteine in hohen Konzentrationen erforderlich. Es hat sich gezeigt, daß pharmazeutisch anwendbare und mit Hedgehog-Protein beschichtete Träger vorzugsweise eine Konzentration des Hedgehog-Proteins von 1 mg/ml Träger und mehr enthalten sollten. Besonders vorteilhaft sind Träger, welche Hedgehog-Proteine in einer Konzentration von 10 mg/ml Träger oder mehr enthalten. Hedgehog-Proteine sind an sich schwer löslich. Es hat sich jedoch überraschenderweise gezeigt, daß in Lösungen die Arginin oder Argininiumionen enthalten, die Löslichkeit von Hedgehog-Proteinen drastisch zunimmt. Ein weiterer Gegenstand der Erfindung sind deshalb erfindungsgemäße wäßrige Lösungen von Hedgehog-Proteinen in einer Konzentration von 3 mg/ml und mehr, welche zusätzlich Arginin oder Argininiumionen enthalten und vorzugsweise gepuffert sind. Ein weiterer Gegenstand der Erfindung ist ein Verfahren zur Herstellung einer mit Hedgehog-Protein beschichteten Trägermatrix, welches dadurch gekennzeichnet ist, daß die Trägermatrix mit einer erfindungsgemäßen Hedgehog-Protein-Lösung in einer Konzentration von 3 mg/ml Hedgehog-Protein, welche die erfindungsgemäßen Zusatzstoffe und Arginin oder Argininiumionen enthält, inkubiert wird und die so beschichtete Trägermatrix isoliert wird.

Solche Lösungen sind geeignet, Trägermatrices herzustellen, welche Hedgehog-Proteine in pharmazeutisch wirksamen Konzentrationen enthalten und für die pharmazeutische Anwendung geeignet sind. Die Konzentration von Arginin ist vorzugsweise zwischen 10 und 100 mmol/l, vorzugsweise im pH-Bereich zwischen 6 und 8.

Unter Aktivität im Sinne der Erfindung ist die Aktivität an alkalischer Phosphatase (Stimulation der Expression der alkalischen Phosphatase) zu verstehen, die das Polypeptid in Säugerzellen induzieren kann (Aktivität im alkalischen Phosphatasetest). Dabei wird eine Maus-Fibroblasten-Zellinie in einem Medium, welches fötales Kälberserum enthält, kultiviert. Anschließend wird sterilfiltrierte Probe zugesetzt, nach ca. 5 Tagen die Zellen aufgeschlossen und in Zellysat alkalische Phosphatase über die Spaltung eines chromogenen Substrats (pNP, p-Nitrophenol) bestimmt (J. Asahina, Exp. Cell. Res. 222 (1996) 38 - 47 und T. Nakamura (1997)).

Unter einem Hedgehog-Protein, gemäß der Erfindung, ist ein sekretiertes Signalprotein zu verstehen, welches für die Ausbildung einer Vielzahl von Strukturen in der Embryogenese verantwortlich ist. Besonders bevorzugt verwendet wird Sonic, Indian- oder Desert hh (Fietz, M., et al., Development (Suppl.) (1994) 43-51). Bevorzugt wird ein hh-Protein einer Sequenz, wie sie in der EMBL-Datenbank unter Nr. L38518 beschrieben ist, verwendet. Proteine der Hedgehog-Familie zeigen eine ausgeprägte Homologie in der Aminosäuresequenz, weshalb es ebenso bevorzugt ist, solche Nukleinsäuren zu exprimieren, welche für Hedgehog-Proteine codieren, die zu 80% oder mehr homolog mit der oben genannten Sequenz von Sonic Hedgehog-Protein sind. Vorzugsweise werden Hedgehog-Derivate verwendet, wie sie beispielsweise in den europäischen Patentanmeldungen Nr. 98102095.1 und 98107911.4 beschrieben sind.

Das humane Sonic Hedgehog-Precursorprotein besteht aus den Aminosäuren 1 - 462 der in der EMBL-Datenbank unter Nr. L38518 beschriebenen Sequenz. Die Aminosäuren 1 - 23 stellen dabei das Signalpeptid dar, die Aminosäuren 24 - 197 die mature Signaldomäne, die Aminosäuren 32 - 197 die um acht Aminosäuren verkürzte Signaldomäne und die Aminosäuren 198 - 462 die autoprozessierte C-terminale Domäne nach autoproteolytischer Spaltung.

Die erfindungsgemäße pharmazeutische Zusammensetzung enthält eine pharmakologisch effektive Dosis des hh-Proteins und kann lokal oder systemisch appliziert werden. Es ist bevorzugt, die erfindungsgemäßen Proteine in Kombination mit anderen Proteinen der Hedgehog-Familie oder Knochenwachstumsfaktoren wie bone morphogenetic proteins (BMPs) (Wozney et al., Cell. Mol. Biol. of Bone, Bone Morphogenetic Proteins and their Gene Expression (1993) Academic Press Inc., 131-167) oder Parathyroidhormonen (Karablis et al., Genes and Development 8 (1994) 277-289) oder insulin-like growth factors (IGF-I oder II) oder transforming growth factors (TGF-β) zu verwenden.

Vorzugsweise enthält die erfindungsgemäße pharmazeutische Zusammensetzung ein Polymeres, welches im wesentlichen als Gerüstsubstanz wirkt, die vorzugsweise auch eine Adhäsionsfunktion für Zellen besitzt. Eine solche Gerüstsubstanz ist beispielsweise Collagen. Dabei ist es bevorzugt, daß die Gerüstsubstanz im Unterschuß zum erfindungsgemäß beschriebenen hydrophilen biokompatiblen Träger ist. Vorzugsweise beträgt der Anteil der Gerüstsubstanz 30% oder weniger, vorzugsweise 10% oder weniger.

Zur Herstellung der pharmazeutischen Zusammensetzung ist es weiter bevorzugt, Hilfsstoffe, wie Zucker (Mannitol, Sucrose, Laktose, Glucose, Saccharose, Trehalose, vorzugsweise 20-100 mg/ml) oder Aminosäuren, wie Glycin oder Arginin, sowie Antioxidantien, wie EDTA, Citrat, Polyethylenglycol (1-10 Gew.%), Detergentien, vorzugsweise nicht-ionische Detergentien (vorzugsweise 0,005-1 Gew.%), wie Polysorbate (Tween® 20 oder Tween® 80) oder Polyoxyethylene, antiinflammatorische Wirkstoffe, lokale Anästhetika, Antibiotika und/oder Stabilisatoren, wie Lipide, Fettsäuren, Glycerin, zuzusetzen.

In einer weiteren bevorzugten Ausführungsform ist eine pharmazeutische Zusammensetzung des erfindungsgemäßen Hedgehog-Proteins mit Suramin bevorzugt und diese vorteilhaft verwendbar.

Die pharmazeutische Zusammensetzung kann weitere pharmazeutische Hilfsstoffe enthalten.

In einer bevorzugten Ausführungsform enthält die pharmazeutische Zusammensetzung Hedgehog-Protein in einer Konzentration von 0,1-100 mg/ml.

In einer bevorzugten Ausführungsform enthält die pharmazeutische Zusammensetzung zusätzlich einen pharmazeutisch akzeptablen Puffer, der bioverträglich ist, vorzugsweise im Bereich zwischen pH 3 und pH 10, besonders bevorzugt im Bereich zwischen pH 6 und 8, insbesondere bei einem pH-Wert von ca. pH 7. Es hat sich überraschenderweise gezeigt, daß die erfindungsgemäßen Zusatzstoffe in der Lage sind, Hedgehog-Proteine auch im sauren Bereich wirksam zu stabilisieren. Der pH-Wert der pharmazeutischen Zusammensetzung sollte zweckmäßig größer pH 4 sein, um eine Denaturierung und die Ablösung des im Hedgehog-Protein komplexierten Zinks zu verhindern. Die Konzentration des Puffers beträgt vorzugsweise 1-500 mmol/l, insbesondere 5 - 150 mmol/l und besonders bevorzugt 10-100 mmol/l. In einer geeigneten Ausführungsform wird als Puffer 20 mmol/l Kaliumphosphatpuffer, pH 7,2 oder 100 mmol/l Argininchlorid, pH 7,2 verwendet.

Die folgenden Beispiele und Publikationen erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

### Beispiel 1

### DSC (Differentielle Scanning Calorimetrie)-Analyse verschiedener hedgehog-Formulierungen

Hedgehog-Proteinlösungen mit einer Proteinkonzentration von ca. 0.5 mg/ml wurden in verschiedenen Puffern (50 mM HEPES-NaOH, pH 7.2 bzw. 150 mM Arginin-Cl, pH 7.4) mit bzw. ohne Stabilisatoren mittels DSC (Nano Differential Scanning Calorimeter, Calorimetry Sciences Corporation, Utah, USA) bei einer Heizrate von 2 K/min analysiert. Als Stabilisatoren wurden verwendet:
- Zinkacetat (Merck)
- Zinksulfat (Merck)
- Heparin (low molecular weight, Sigma)
- sulfatiertes β-Cyclodextrin (Aldrich)

Die für die jeweiligen Formulierungen ermittelten Übergangstemperaturen (Tt) sind in der Tabelle zusammengefaßt. Aus den aufgeführten Daten wird ersichtlich, daß durch Zugabe der im Text erwähnten Substanzen die Übergangstemperatur und damit die Stabilität des hedgehog-Proteins erhöht wird. Die gemessenen Temperaturwerte sind nicht als Absolutwerte zu verstehen, sondern stellen die Unterschiede der Stabilität der einzelnen Formulierungen relativ zueinander dar.

| **Formulierung** | **Tt [°C]** |
|---|---|
| 50 mM Hepes-NaOH, pH 7.2 | 52.0 |
| 50 mM Hepes-NaOH, 1 mM Zinkacetat, pH 7.2 | 56.8 |
| 50 mM Hepes-NaOH, 1 mM Zinksulfat, pH 7.2 | 60.6 |
| 150 mM Argininchlorid, pH 7.4 | 53. 5 |
| 150 mM Argininchlorid, 5 % (w/v) sulfatiertes β-Cyclodextrin, pH 7.4 | 55.6 |
| 150 mM Argininchlorid, 20 mg/ml Heparin, pH 7.4 | 57.8 |

Übergangstemperaturen für hedgehog-Proteine in unterschiedlichen Formulierungen

### Referenzliste

Asahina, J., Exp. Cell. Res. 222 (1996) 38-47
Bitgood, M.J., et al., Curr. Biol. 6 (1996) 296
Chiang, C., et al., Nature 83 (1996) 407
Europäische Patentanmeldung Nr. 98102095.1
Europäische Patentanmeldung Nr. 98107911.4
Fietz, M., et al., Development (Suppl.) (1994) 43-51
Hynes, M., et al., Neuron 15 (1995) 35-44
Karablis et al., Genes and Development 8 (1994) 277-289
Kinto et al., FEBS Letters, 404 (1997) 319-323
Lai, C.J., et al., Development 121 (1995) 2349
Lopez-Martinez et al., Curr. Biol. 5 (1995) 791-796
Marti et al., Nature 375 (1995) 322-325
Miao et al., J. Neurosci. 17 (1997) 5891-5899
Nakamura, T., et al., Biochem. Biophys. Res. Comm. 237 (1997) 465-469
Pepinski et al., J. Biol. Chem. 273 (1998) 14037 - 14045
Perrimon, N., Cell 80 (1995) 517-520
Porter, J.A., et al., Science 274 (1996) 255-259
Smith, J.C., Cell 76 (1994) 193-196
Stott et al., J. Cell Sci. 110 (1997) 2691-2701
Vortkamp, A., et al., Science 273 (1996) 613
Wozney et al., Cell. Mol. Biol. of Bone, Bone Morphogenetic Proteins and their Gene Expression (1993) Academic Press inc., 131-167
Yang et al., Development 124 (1997) 4393-4404

## Patentansprüche

1. Pharmazeutische Zusammensetzung eines Hedgehog-Proteins, welche ein Hedgehog-Protein in einer pharmazeutisch wirksamen Menge sowie als Zusatzstoff Zinkionen, Sulfationen, Magnesiumionen, Calciumionen, Sulfationen, Cyclodextrin und/oder Heparin enthält.

2. Pharmazeutische Zusammensetzung nach Anspruch 1, dadurch gekennzeichnet, daß diese Zusammensetzung Zinksulfat enthält.

3. Pharmazeutische Zusammensetzung nach Anspruch 1, dadurch gekennzeichnet, daß diese Zusammensetzung Cyclodextrinsulfat enthält.

4. Pharmazeutische Zusammensetzung nach den Ansprüchen 1 oder 2, dadurch gekennzeichnet, daß diese Zusammensetzung Zinkionen in einer Konzentration von 0,01 - 100 mmol/l enthält.

5. Pharmazeutische Zusammensetzung nach den Ansprüchen 1 - 4, dadurch gekennzeichnet, daß das Hedgehog-Protein an einen hydrophilen Träger, der biokompatibel ist, gebunden ist.

6. Pharmazeutische Zusammensetzung nach den Ansprüchen 1 - 5, dadurch gekennzeichnet, daß der hydrophile Träger ein Polymer ist.

7. Pharmazeutische Zusammensetzung nach den Ansprüchen 1 - 6, dadurch gekennzeichnet, daß die pharmazeutische Zusammensetzung ein Hedgehog-Protein in einer Konzentration von 0,1-100 mg/ml enthält.

8. Pharmazeutische Zusammensetzung nach den Ansprüchen 1 - 7, dadurch gekennzeichnet, daß die Zusammensetzung im Bereich zwischen pH 3 und 10 gepuffert ist.

9. Pharmazeutische Zusammensetzung nach den Ansprüchen 1 - 8, dadurch gekennzeichnet, daß das Hedgehog-Protein an einen biokompatiblen Träger gebunden ist.

10. Verfahren zur Herstellung einer pharmazeutischen Zusammensetzung, welche ein Hedgehog-Protein enthält, dadurch gekennzeichnet, daß als wesentliche Bestandteile dieses Mittels ein Hedgehog-Protein in einer pharmazeutischen Menge sowie als Zusatzstoff Zinkionen, Magnesiumionen, Calciumionen, Sulfationen, Cyclodextrin und/oder Heparin verwendet wird.

11. Verfahren nach Anspruch 10, dadurch gekennzeichnet, daß ein Hedgehog-Protein in einer Konzentration von 0,1-100 mg/ml verwendet wird.

12. Verfahren zur verzögerten Freisetzung eines Hedgehog-Proteins im menschlichen Körper, dadurch gekennzeichnet, daß das Hedgehog-Protein in einer pharmazeutischen Zusammensetzung nach den Ansprüchen 1 bis 9 lokal im menschlichen Körper appliziert wird.

13. Verfahren nach Anspruch 12, dadurch gekennzeichnet, daß das Hedgehog-Protein in einer Konzentration von 0,1-100 mg/ml appliziert wird.

14. Wässrige Zusammensetzung eines Hedgehog-Proteins, dadurch gekennzeichnet, daß diese Zusammensetzung einen oder mehrere Zusatzstoffe ausgewählt aus der Gruppe Zinkionen, Magnesiumionen, Calciumionen, Sulfationen, Cyclodextrin oder Heparin in einem molaren Überschuß gegenüber dem Hedgehog-Protein enthält.

15. Lyophilisat einer pharmazeutischen Zusammensetzung nach den Ansprüchen 1 - 9 oder einer wässrigen Zusammensetzung nach Anspruch 14.
